# EUROPEAN PATENT SPECIFICATION

(11) **EP 4 241 585 B1**
(45) Date of publication and mention of the grant of the patent: **13.05.2026**
(21) Application number: 23155264.7
(22) Date of filing: 07.02.2023
(51) Int. Cl.: A24F 15/015, A24F 40/00, A61M 11/04, A61M 15/00, B05B 17/06, A24F 40/42, A24F 40/49

(54) **ATOMIZING DEVICE**
ZERSTÄUBUNGSVORRICHTUNG
DISPOSITIF DE PULVERISATION

(30) Priority: 11.03.2022 CN 202210241350
(43) Date of publication of application: 13.09.2023
(73) Proprietor: Shenzhen Merit Technology Co., Ltd., Shenzhen, Guangdong 518000 (CN)
(72) Inventor: WANG, Wuchao, Shenzhen, Guangdong, 518000 (CN); TANG, Yuxiang, Shenzhen, Guangdong, 518000 (CN); LIAO, Yancheng, Shenzhen, Guangdong, 518000 (CN); WANG, Wei, Shenzhen, Guangdong, 518000 (CN)
(74) Representative: Michalski Hüttermann & Partner Patentanwälte mbB

(56) References cited:
- EP-A1- 3 498 113
- EP-A1- 3 536 175
- WO-A1-2021/140317
- GB-A- 2 533 874
- US-A1- 2016 150 824
- US-A1- 2019 216 134

## Description

### TECHNICAL FIELD

The present disclosure relates to a technical field of atomization, in particular to an atomizing device.

### BACKGROUND

Aerosol is a colloidal dispersion system formed by small solid or liquid particles dispersed and suspended in a gas medium. Since the aerosol can be absorbed by a human body through a respiratory system, it provides users with a new alternative absorption method. An atomizing device is configured to atomize an atomizing medium by heating or ultrasound to generate the aerosol. The atomizing medium include nicotine-containing e-liquid, medical drugs, skin care lotions, etc. The atomizing medium is atomized to generate the aerosol that can be inhaled as an alternative to conventional inhaled product.

The existing atomizing devices can be divided into closed-type atomizing devices and open-type atomizing devices according to the way of liquid injecting. The atomizing medium of the closed-type atomizing device is filled during the production and cannot be refilled after use, so the cost is higher for the user. The open-type atomizing device is provided with a liquid injecting hole on a shell thereof and sealed with a sealing element. After the atomizing medium in the atomizing device is used up, the sealing element can be provided to refill atomizing device with the atomizing medium, so the open-type atomizing device can be reused, thereby reducing the cost and more environmentally friendly. However, the sealing element of the open-type atomizing device is easy to open. If a child opens the sealing element, it is easy to cause leakage and there is a risk of eating the atomizing medium by the child. WO 2021/140317 A1 discloses a atomizing device with a body and a changeable cartridge having a chamber, wherein the chamber is refillable via openings. EP 3 498 113 A1 discloses an electronic smoking device with an atomizer/liquid reservoir portion. In order to facilitate refilling a liquid reservoir, the atomizer/liquid reservoir portion comprises a docking valve for docking a source of liquid to be stored in the liquid reservoir, wherein the docking valve is adapted to form a snap-fit connection with a counter docking valve of a source of liquid.

### SUMMARY

Accordingly, an atomizing device according to claim 1 is provided, which can prevent children from opening a liquid injecting hole and causing leakage and accidental ingestion liquid.

An atomizing device includes a liquid storing element provided with a liquid injecting hole, a sealing element detachably connected to the liquid storing element and configured to seal the liquid injecting hole, and a protecting element connected to the liquid storing element. The protecting element is movable relative to the liquid storing element and is capable of switching between a first position and a second position, wherein the shielding area of the sealing element by the protecting element in the first position is greater than the shielding area of the sealing element by the protecting element in the second position.

In one of the embodiments, at least part of the sealing element is shielded by the protecting element when the protecting element is in the first position, the sealing element is completely exposed to external environment when the protecting element is in the second position.

The protecting element is capable of moving along the axial direction of the liquid storing element relative to the liquid storing element.

When the protecting element is in the first position, the protecting element is capable of rotating around the axial direction of the liquid storing element and is capable of switching between a locking state and an unlocking state; when the protecting element is in the locking state, the protecting element is fixed relative to the liquid storing element in the axial direction of the liquid storing element; when the protecting element is in the unlocking state, the protecting element is capable of moving in the axial direction of the liquid storing element to be switched to the second position.

In one of the embodiments, one of the liquid storing element and the protecting element is provided with a buckle, the other one of the liquid storing element and the protecting element is provided with a first guiding groove extending along the circumferential direction of the liquid storing element, when the protecting element is in the locking state, the buckle is engaged in the first guiding groove.

In one of the embodiments, a transiting portion protrudes within the first guiding groove, when the protecting element is in the unlocking state, the buckle abuts against the transiting portion to be disengaged from the first guiding groove.

In one of the embodiments, one of the liquid storing element and the protecting element provided with the first guiding groove is further provided with a second guiding groove, the second guiding groove is located on a side of the first guiding groove in the axial direction of the liquid storing element, when the protecting element is in the second position, the buckle is limited in the second guiding groove.

In one of the embodiments, the second guiding groove and the first guiding groove are spaced apart in the axial direction of the liquid storing element; or
the second guiding groove is in communication with the first guiding groove, and a bottom wall of the second guiding groove is smoothly connected to an outer surface of the transiting portion.

In one of the embodiments, the protecting element is provided with an indicating groove, when the protecting element is in the locking state, the indicating groove and the sealing element are misaligned in the circumferential direction of the liquid storing element, when the protecting element is in the unlocking state, the indicating groove is aligned with the sealing element in the circumferential direction of the liquid storing element.

In one of the embodiments, one of the liquid storing element and the protecting element is provided with an engaging protrusion, the other one of the liquid storing element and the protecting element is provided with an engaging groove and a sliding groove, the engaging groove and the sliding groove are spaced apart in the circumferential direction of the liquid storing element, and the length of the sliding groove is greater than the length of the engaging groove in the axial direction of the liquid storing element, when the protecting element is in the locking state, the engaging protrusion is engaged in the engaging groove, when the protecting element is in the unlocking state, the engaging protrusion is located in the sliding groove.

In one of the embodiments, an attractive force is provided between the liquid storing element and the protecting element, when the protecting element is in the first position, the liquid storing element and the protecting element are attracted to each other, when the protecting element is in the second position, the protecting element is disengaged from the liquid storing element by an external force that overcomes the attractive force.

In one of the embodiments, one of the liquid storing element and the protecting element is provided with a limiting groove, the other one of the liquid storing element and the protecting element is provided with a limiting block, the limiting groove extends in the axial direction of the liquid storing element or the protecting element, the limiting block extends into the limiting groove and is capable of sliding along the limiting groove.

In one of the embodiments, the protecting element is rotatable relative to the liquid storing element about the axial direction of the liquid storing element.

In one of the embodiments, the protecting element is provided with a liquid injecting notch, when the protecting element is in the first position, the liquid injecting notch and the sealing element are misaligned, when the protecting element is in the second position, the liquid injecting notch is aligned with the sealing element to expose the sealing element.

In one of the embodiments, one of the liquid storing element and the protecting element is provided with at least two engaging notches, the at least two engaging notches are spaced apart in the circumferential direction of the protecting element, the other one of the liquid storing element and the protecting element is provided with an engaging portion, the engaging portion is engaged in one of the engaging notches when the protecting element is in the first position, and the engaging portion is engaged in another engaging notch when the protecting element is rotated to the second position.

The atomizing device can effectively prevent the child from opening the liquid injecting hole to contact or even accidentally eat the atomizing medium though the protecting element, which improves the safety performance of the atomizing device, effectively protects the health of children and prevents the waste of the atomizing medium.

### BRIEF DESCRIPTION OF THE DRAWINGS

The above and other objects and features of the present disclosure will become apparent and more readily appreciated from the following description of the embodiments with reference to the following figures, wherein like reference numerals refer to like parts throughout the various figures unless otherwise specified.
FIG. 1 is a perspective view of an atomizing device according to a first embodiment of the present disclosure when a protecting element is in a first position and in a locking state.
FIG. 2 is similar to FIG. 1, but shows the protecting element in an unlocking state.
FIG. 3 is similar to FIG. 2, but shows the protecting element in a second position.
FIG. 4 is an exploded view of the atomizing device shown in FIG. 1.
FIG. 5 is a front view of the atomizing device shown in FIG. 1.
FIG. 6 is a cross-sectional view of the atomizing device taken along the line A-A shown in FIG. 5.
FIG. 7 is a perspective view of an atomizing device according to a second embodiment of the present disclosure when a protecting element is in a first position and in a locking state.
FIG. 8 is similar to FIG. 7, but shows the protecting element in an unlocking state.
FIG. 9 is similar to FIG. 8, but shows the protecting element in a second position.
FIG. 10 is an exploded view of the atomizing device shown in FIG. 7.
FIG. 11 is a perspective view of an atomizing device according to a third embodiment of the present disclosure when a protecting element is in a first position.
FIG. 12 is similar to FIG. 11, but shows the protecting element in a second position.
FIG. 13 is an exploded view of the atomizing device shown in FIG. 11.
FIG. 14 is a perspective view of an atomizing device according to a fourth embodiment of the present disclosure when a protecting element is in a first position.
FIG. 15 is similar to FIG. 14, but viewed from another aspect.
FIG. 16 is similar to FIG. 14, but shows the protecting element in a second position.
FIG. 17 is an exploded view of the atomizing device shown in FIG. 14.

Description of reference numerals:
100, atomizing device; 120, liquid storing element; 121, sealing element; 123, first guiding groove; 125, transiting portion; 127, second guiding groove; 140, protecting element; 141, buckle; 143, indicating groove; 200, atomizing device; 210, liquid storing element; 212, sealing element; 214, engaging protrusion; 230, protecting element; 232, engaging groove; 234, sliding groove; 300, atomizing device; 320, liquid storing element; 321, sealing element; 323, attracting element; 325, limiting block; 340, protecting element; 341, limiting groove; 400, atomizing device; 410, liquid storing element; 412, sealing element; 414, engaging portion; 430, protecting element; 432, liquid injecting notch; 434, engaging notch.

### DETAILED DESCRIPTION OF THE EMBODIMENTS

In order to make the above objects, features and advantages of the present disclosure more obvious and easier to understand, the specific embodiments of the present disclosure are described in detail below in combination with the accompanying drawings. Many specific details are set forth in the following description to facilitate a full understanding of the invention. However, the present disclosure can be implemented in many ways different from those described herein, and those skilled in the art can make similar improvements without violating the connotation of the invention. Therefore, the invention is not limited by the specific embodiments disclosed below.

In the description of the present disclosure, it should be understood that the terms "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential direction" are based on the azimuth or position relationship shown in the attached drawings, which is only for the convenience of describing the present disclosure and simplifying the description, rather than indicating or implying that the device or element must have a specific azimuth, be constructed and operated in a specific azimuth, so it cannot be understood as a limitation of the present disclosure.

In addition, the terms "first" and "second" are only used for descriptive purposes and cannot be understood as indicating or implying relative importance or implicitly indicating the number of indicated technical features. Thus, the features defined with "first" and "second" may explicitly or implicitly include at least one of the features. In the description of the present disclosure, "multiple" means at least two, such as two, three, etc., unless otherwise expressly and specifically defined.

In the present disclosure, unless otherwise expressly specified and limited, the terms "install", "connect", "contact", "fix" and other terms should be understood in a broad sense, for example, they can be fixed connections, removable connections, or integrated. It can be mechanical connection or electrical connection. It can be directly connected or indirectly connected through an intermediate medium. It can be the connection within two elements or the interaction relationship between two elements, unless otherwise expressly limited. For those skilled in the art, the specific meaning of the above terms in the present disclosure should be understood according to the specific situation.

In the present disclosure, unless otherwise expressly specified and limited, the first feature "above" or "below" the second feature may be in direct contact with the first and second features, or the first and second features may be in indirect contact through an intermediate medium. Moreover, the first feature is "above" the second feature, but the first feature is directly above or diagonally above the second feature, or it only means that the horizontal height of the first feature is higher than the second feature. The first feature is "below" of the second feature, which can mean that the first feature is directly below or obliquely below the second feature, or simply that the horizontal height of the first feature is less than that of the second feature.

It should be noted that when an element is called "fixed to" or "disposed on" another element, it can be directly on another element or there can be a centered element. When an element is considered to be "connected" to another element, it can be directly connected to another element or there may be intermediate elements at the same time. The terms "vertical", "horizontal", "up", "down", "left", "right" and similar expressions used herein are for the purpose of illustration only and do not represent the only embodiment.

An atomizing device is provided according to an embodiment of the present disclosure, which is configured to heat an atomizing medium to generate an aerosol for human inhalation. The atomizing device includes a liquid storing element and a sealing element. The liquid storing element is provided with a liquid injecting hole for injecting the atomizing medium, the sealing element is detachably connected to the liquid storing element, and the sealing element is configured to seal the liquid injecting hole. When injecting the atomizing medium, a user can open the sealing element to expose the liquid injecting hole to inject the atomizing medium. When there is no need to inject the atomizing medium, the liquid injecting hole can be sealed by the sealing element to prevent the leakage of the atomizing medium through the liquid injecting hole.

In order to prevent a child from easily opening the liquid injecting hole and contacting the atomizing medium, the atomizing device further includes a protecting element. The protecting element is movably connected to the liquid storing element, and is capable of switching between a first position and a second position. The shielding area of the sealing element by the protecting element in the first position is greater than the shielding area of the sealing element by the protecting element in the second position, so as to increase the difficulty of opening the liquid injecting hole. Specifically, in an embodiment, when the protecting element is in the first position, at least part of the sealing element is shielded by the protecting element, so that the child cannot open the liquid injecting hole. When the protecting element is in the second position, the sealing element is completely exposed to the external environment, so the user can easily open the liquid injecting hole to inject the atomizing medium. In other embodiments, when the protecting element is in the second position, the sealing element may also be partially shielded by the protecting element, but the shielded area is reduced, the user can operate to open the liquid injecting hole, which is not limited herein.

In this way, the protecting element can effectively prevent the child from opening the liquid injecting hole to contact or even accidentally eat the atomizing medium, which improves the safety performance of the atomizing device, effectively protects the health of children and prevents the waste of the atomizing medium.

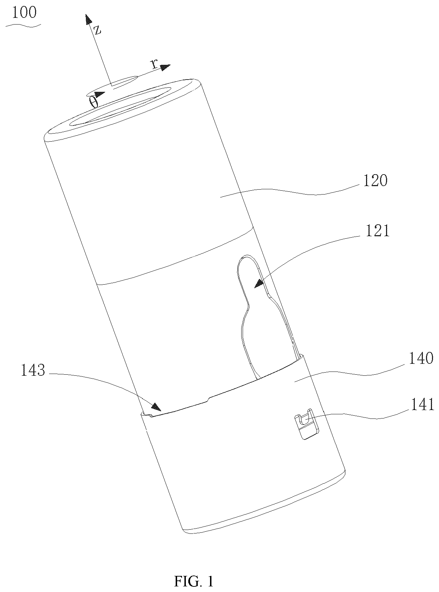

It should be noted that, in the present disclosure, the axial direction of the liquid storing element and the protecting element is the same as a central axis thereof, a radial direction of the liquid storing element and the protecting element is perpendicular to the axial direction. That is, the liquid storing element and the protecting element are perpendicular to a radius direction of the cross-section of the central axis, the circumferential direction of the liquid storing element and the protecting element surrounds the central axis and perpendicular to the axial direction and the radial direction. For example, as shown in FIG. 1, the axial direction of the liquid storing element is represented as z, the radial direction of the liquid storing element is represented as r, the circumferential direction of the liquid storing element is represented as θ.

As shown in FIG. 1 to FIG. 3, an atomizing device 100 is provided according to a first embodiment of the present disclosure, which includes a liquid storing element 120 and a protecting element 140. The liquid storing element 120 is substantially cylindrical, and is provided with a liquid injecting hole (not shown) for injecting the atomizing medium. The atomizing device 100 further includes a sealing element 121 provided in an axial middle portion of the liquid storing element 120. The sealing element 121 is configured to seal the liquid injecting hole. The protecting element 140 has a hollow cylindrical structure and is sleeved on the liquid storing element 120, and a central axis of the protecting element 140 is coincident with a central axis of the liquid storing element 120. The protecting element 140 is capable of moving along the axial direction of the liquid storing element 120 relative to the liquid storing element 120, so as to be switched between a first position and a second position.

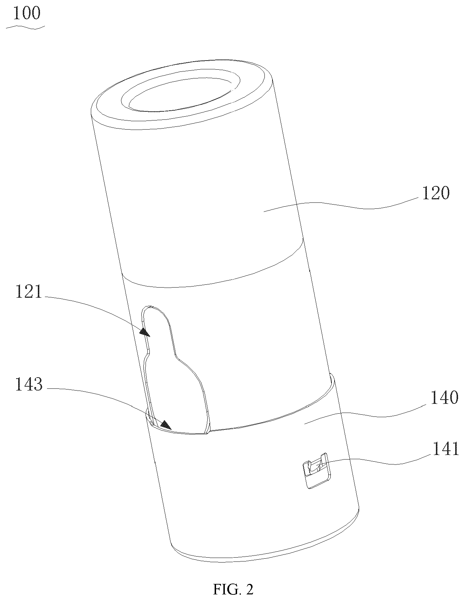

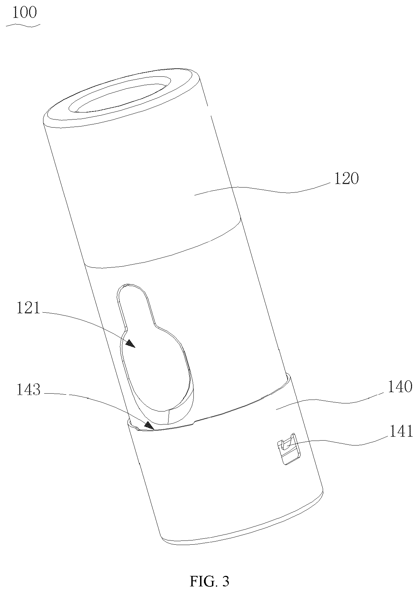

As shown in FIGS. 1 and 2, when the protecting element 140 is in the first position, the protecting element 140 is capable of rotating around the axial direction of the liquid storing element 120 and is capable of switching between a locking state and an unlocking state. Further referring to FIG. 3, when the protecting element 140 is in the locking state (as shown in FIG. 1), the protecting element 140 is fixed relative to the liquid storing element 120 in the axial direction of the liquid storing element 120, so that the child cannot push the protecting element 140 along the axial direction of the liquid storing element 120 to switch the protecting element 140 to the second position. When the protecting element 140 is in the unlocking state (as shown in FIG. 2), the protecting element 140 can move in the axial direction of the liquid storing element 120 to be switched to the second position (as shown in FIG. 3).

In this way, the protecting element 140 in the locking state can be kept in the first position. When the atomizing medium needs to be refilled, the protecting element 140 needs to be switched to the unlocking state before moving to the second position, thus further improving the protective performance of the protecting element 140, thereby enhancing the safety of the atomizing device 100.

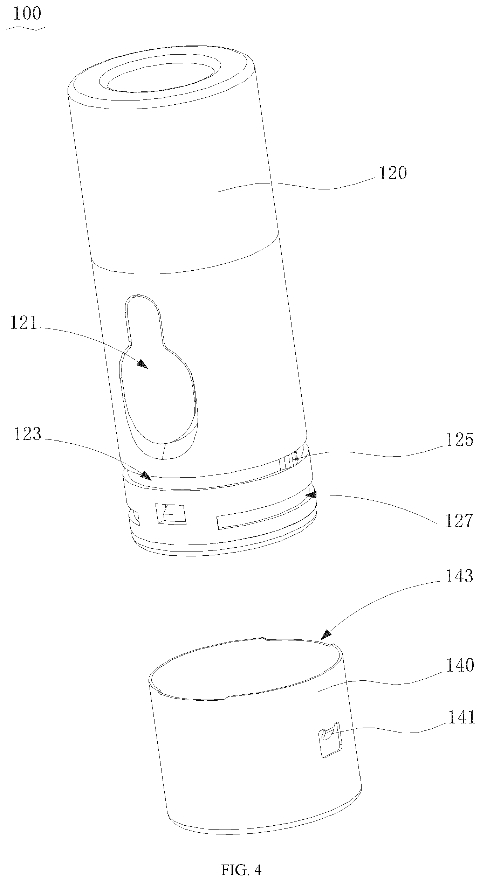

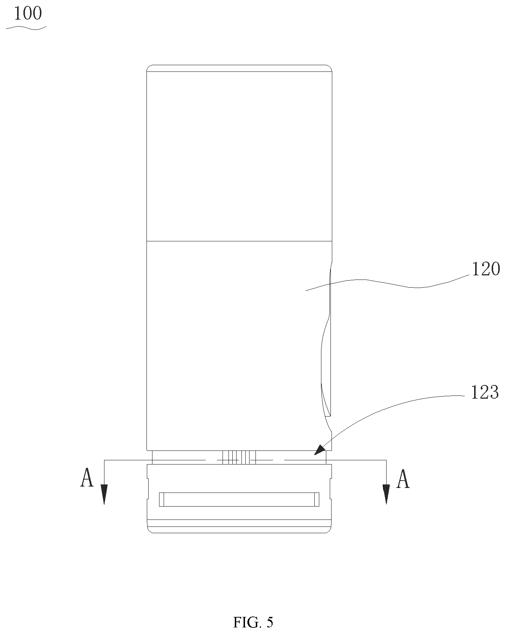

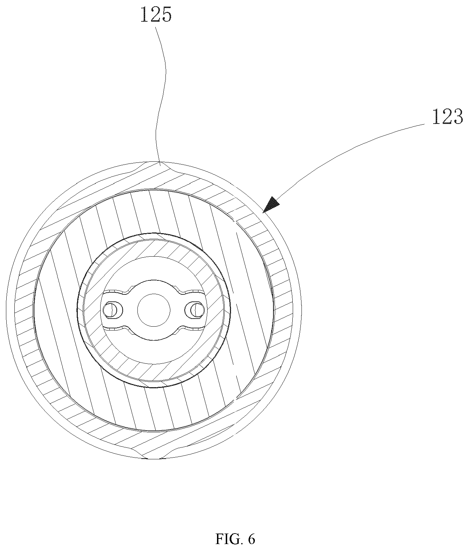

Referring to FIG. 4 to FIG. 6, the protecting element 140 is provided with two buckles 141. The two buckles 141 are radially located on opposite sides of the protecting element 140, respectively, each buckle 141 is bent toward an inner side of the protecting element 140 to protrude from the inner surface of the protecting element 140. The liquid storing element 120 is provided with a first guiding groove 123 located on one side of the sealing element 121 in the axial direction thereof, and the first guiding groove 123 extends in a circular shape along the circumferential direction of liquid storing element 120. When the protecting element 140 is in the locking state, the buckle 141 is engaged in the first guiding groove 123. Under the limiting effect of the first guiding groove 123, the protecting element 140 cannot move in the axial direction of the liquid storing element 120 and is kept in the first position.

In order to enable the protecting element 140 to be moved to the second position, two transiting portions 125 protrude within the first guiding groove 123. The two transiting portions 125 are respectively located on opposite sides in the radial direction of the liquid storing element 120 to correspond to the two buckles 141, and an edge of each transiting portion 125 is smoothly connected to a bottom wall of the first guiding groove 123. When the protecting element 140 rotates around the axial direction of the liquid storing element 120, the protecting element 140 can move to the unlocking state along the first guiding groove 123. At this time, the buckle 141 is elastically deformed under the resisting action of the transiting portion 125 to be disengaged from the first guiding groove 123. After releasing the limiting effect of the first guiding groove 123, the protecting element 140 can move relative to the liquid storing element 120 along the axial direction of the liquid storing element 120 to be switched to the second position. It should be understood that, in other embodiments, after the protecting element 140 is switched to the second position, the protecting element 140 can be continued to move to completely disengage the protecting element 140 from the liquid storing element 120.

Further, in some embodiments, in order to prevent the protecting element 140 in the second position from being separated from the liquid storing element 120 and lost, the liquid storing element 120 is further provided with a second guiding groove 127. The second guiding groove 127 is located on a side of the first guiding groove 123 in the axial direction of the liquid storing element 120 and extends along the circumferential direction of the liquid storing element 120. When the protecting element 140 is in the second position, the buckle 141 restores its initial shape and is engaged in the second guiding groove 127, so that the protecting element 140 cannot continue to move in the axial direction of the liquid storing element 120, thereby limiting the protecting element 140 in the second position.

Specifically, in an embodiment, the second guiding groove 127 and the first guiding groove 123 are spaced apart in the axial direction of the liquid storing element 120. In another embodiment, the second guiding groove 127 is in communication with the first guiding groove 123, and a depth of the second guiding groove 127 in the radial direction of the liquid storing element 120 is less than a depth of the first guiding groove 123 in the radial direction of the liquid storing element 120. A bottom wall of the second guiding groove 127 is smoothly connected to an outer surface of the transiting portion 125. In this way, the buckle 141 abutting against the transiting portion 125 can smoothly slide into the second guiding groove 127, then the protecting element 140 and the liquid storing element 120 are prevented from being separated by the limiting effect of the second guiding groove 127.

As shown in FIG. 1, FIG. 2 and FIG. 4, in some embodiments, an edge of the protecting element 140 facing the sealing element 121 is provided with an indicating groove 143. The indicating groove 143 extends longitudinally along the circumferential direction of the protecting element 140, the length of the indicating groove 143 matches a width of the sealing element 121. When the protecting element 140 is in the locking state, the indicating groove 143 and the sealing element 121 are misaligned in the circumferential direction of the liquid storing element 120. When the protecting element 140 is in the unlocking state, the indicating groove 143 is aligned with the sealing element 121 in the circumferential direction of the liquid storing element 120. In this way, the user can quickly identify the state of the protecting element 140 according to the position of the indicating groove 143.

It should be understood that the arrangement positions of the first guiding groove 123, the second guiding groove 127, and the buckle 141 are not limited hereto. In some other embodiments, the first guiding groove 123 and the second guiding groove 127 are both provided on an inner sidewall in the protecting element 140, the buckle 141 is provided on an outer sidewall of the liquid storing element 120, which can also realize the switching of the protecting element 140 between the first position and the second position, the locking state and the unlocking state.

A working principle of the atomizing device 100 will be described as follows.

As shown in FIG. 1, when there is no need to add the atomizing medium, the indicating groove 143 and the sealing element 121 are misaligned in the circumferential direction of the liquid storing element 120, the buckle 141 is engaged in the first guide groove 123. At this time, the protecting element 140 is in the locking state, the protecting element 140 shields at least part of the sealing element 121 to prevent the child from opening the liquid injecting hole.

As shown in FIG. 2, when adding the atomizing medium, the user can rotate the protecting element 140 to enable the indicating groove 143 to be aligned with the sealing element 121 of the liquid storing element 120, and the buckle 141 on the protecting element 140 is elastically deformed against the transiting portion 125 and disengaged from the first guide groove 123 to be in an unlocking state. Then the user can pull the protecting element 140 along the axial direction of the liquid storing element 120 to move the protecting element 140 to the second position (as shown in FIG. 3), the buckle 141 is engaged in the second guiding groove 127. At this time, the sealing element 121 is completely exposed to the external environment, the user can open the sealing element 121 to add the atomizing medium through the liquid injecting hole.

After the atomizing medium is added, the protecting element 140 can be pushed along the axial direction of the liquid storing element 120, the buckle 141 is disengaged from the second guiding groove 127 and abuts against the transiting portion 125 again, then the protecting element 140 is rotated to enable the buckle 141 to be engaged back into the first guiding groove 123.

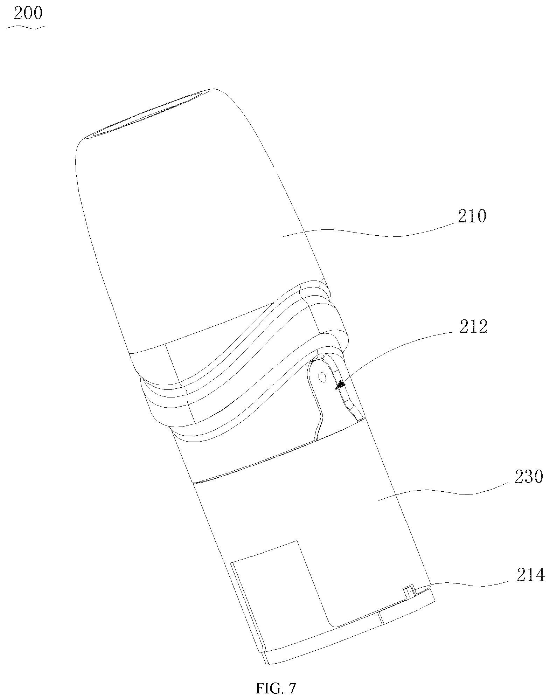

As shown in FIG. 7 to FIG. 9, an atomizing device 200 is provided according to a second embodiment of the present disclosure, which includes a liquid storing element 210 and a protecting element 230. An end of the liquid storing element 210 provided with the sealing element 212 is substantially cylindrical, the protecting element 230 has a hollow cylindrical structure and is sleeved on the liquid storing element 210, and a central axis of the protecting element 230 is coincident with a central axis of the liquid storing element 210. The protecting element 230 is movable relative to the liquid storing element 210 in the axial direction of the liquid storing element 210 to be switched between a first position and a second position.

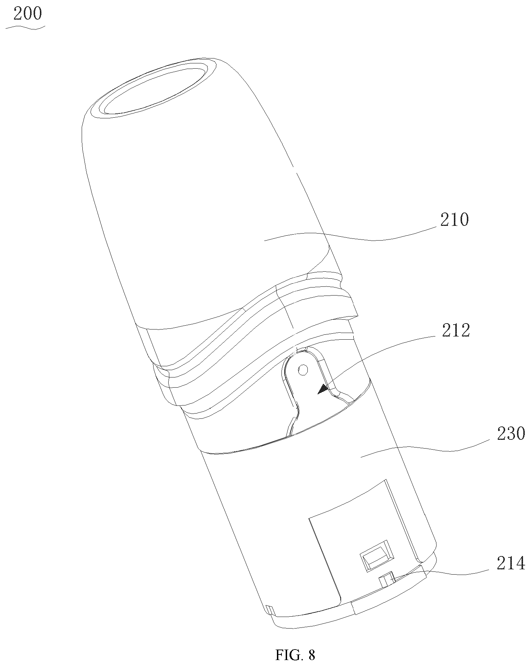

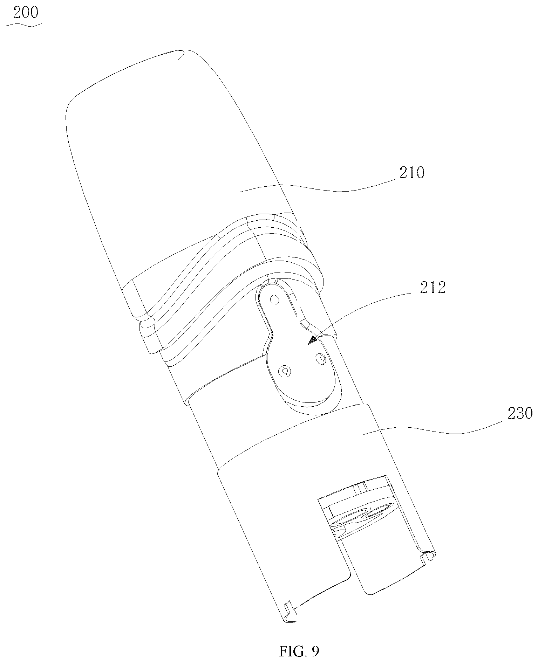

As shown in FIGS. 7 and 8, when the protecting element 230 is in the first position, the protecting element 230 can be rotated around the axial direction of the liquid storing element 210 to be switched between a locking state and an unlocking state. When the protecting element 230 is in the locking state, the protecting element 230 is fixed relative to the liquid storing element 210 in the axial direction of the liquid storing element 210, such that the child cannot switch the protecting element 230 to the second position to expose the sealing element 212. As shown in FIGS. 8 and 9, when the protecting element 230 is in the unlocking state, the protecting element 230 can move along the axial direction of the liquid storing element 210 to be switched to the second position to expose the sealing element 212.

In this way, the protecting element 230 in the locking state can be kept in the first position. When the atomizing medium needs to be added, the protecting element 230 can be switched to the unlocking state before moving to the second position, thus further improving the protective performance of the protecting element 230, thereby enhancing the safety of the atomizing device 200.

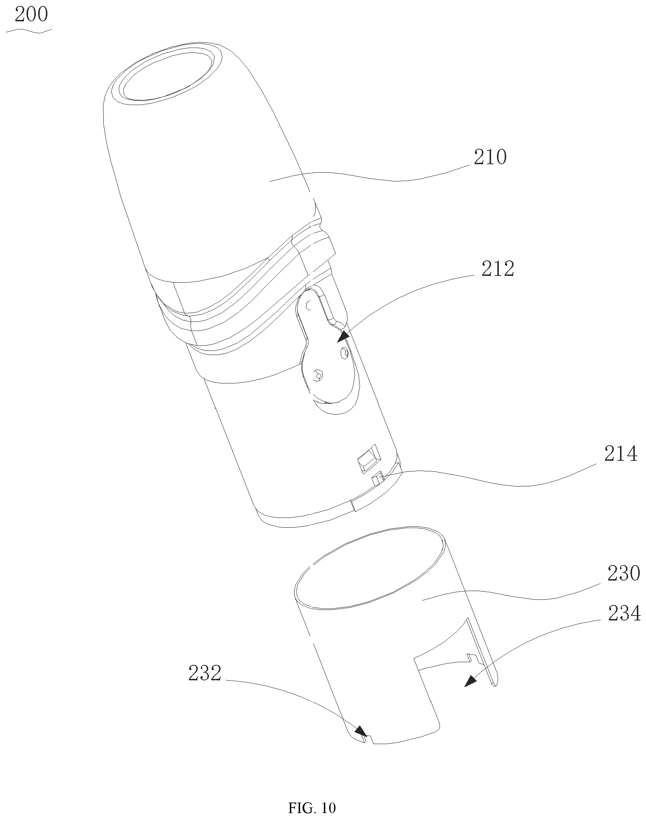

Referring to FIG. 10, specifically, an outer sidewall of a bottom of the liquid storing element 210 is provided with at least one engaging protrusion 214, an end of the protecting element 230 is provided with at least one engaging groove 232 and at least one sliding groove 234. The engaging groove 232 and the sliding groove 234 are spaced apart in the circumferential direction of the liquid storing element 210, and the length of the sliding groove 234 is greater than the length of the engaging groove 232 in the axial direction of the liquid storing element 210. A shape of the engaging groove 232 matches a shape of the engaging protrusion 214, the sliding groove 234 extends longitudinally from one end of the protecting element 230 toward the other end thereof to form a long strip.

As shown in FIG. 7, when the protecting element 230 is in the locking state, the engaging protrusion 214 is engaged in the engaging groove 232, and the engaging groove 232 is configured to limit the movement of the protecting element 230 along the axial direction of the liquid storing element 210. As shown in FIG. 8, when the protecting element 230 is rotated to be elastically deformed to be switched to the unlocking state, the engaging protrusion 214 is disengaged from the engaging groove 232 to be located in the sliding groove 234. Since the sliding groove 234 extends longitudinally along the axial direction of the liquid storing element 210, the engaging protrusion 214 can slide along the sliding groove 234, so that the protecting element 230 can slide relative to the liquid storing element 210 along the axial direction of the liquid storing element 210 to be switched to the second position (as shown in FIG. 9).

In some other embodiments, the engaging protrusion 214 is provided on the protecting element 230, the engaging groove 232 and the sliding groove 234 are provided on the liquid storing element 210, which can also realize the switching of the protecting element 230 between the first position and the second position, the locking state and the unlocking state.

A working principle of the atomizing device 200 will be described as follows.

As shown in FIG. 7, when there is no need to add the atomizing medium, the engaging protrusion 214 is engaged in the engaging groove 232, at this time the protecting element 230 is in the locking state, the protecting element 230 shields at least part of the sealing element 212 to prevent the child from opening the liquid injecting hole.

As shown in FIG. 8 and FIG. 9, when adding the atomizing medium, the protecting element 230 is rotated to generate a recoverable deformation until the engaging protrusion 214 is engaged in the sliding groove 234. Since the sliding groove 234 extends longitudinally along the axial direction of the liquid storing element 210, the protecting element 230 can be pulled to move to the second position along the axial direction of the liquid storing element 210. At this time, the sealing element 212 is completely exposed to the external environment, and the operator can open the sealing element 212 to add the atomizing medium through the liquid injecting hole.

After the atomizing medium is added, the protecting element 230 is pushed along the axial direction of the liquid storing element 210 to the first position, then the protecting element 230 is rotated to generate a recoverable deformation until the engaging protrusion 214 is engaged in the engaging groove 232 again.

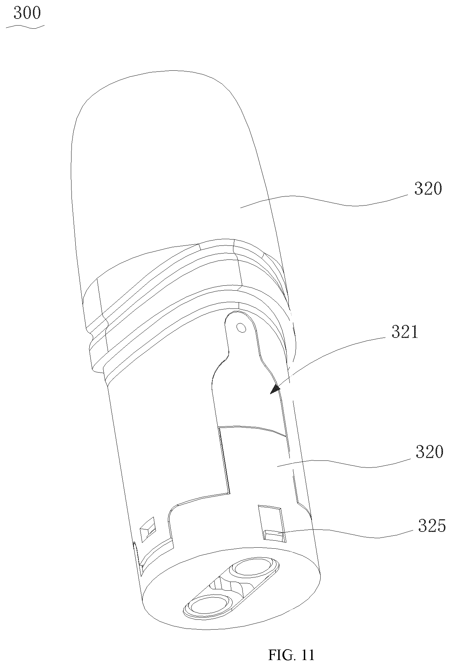

As shown in FIG. 11 to FIG. 13, an atomizing device 300 is provided according to a third embodiment of the present disclosure, which includes a liquid storing element 320 and a protecting element 340. An end of the liquid storing element 320 provided with the sealing element 321 is substantially cylindrical, the protecting element 340 a hollow cylindrical structure and is sleeved on the liquid storing element 320, and a central axis of the protecting element 340 is coincident with a central axis of the liquid storing element 320. The protecting element 340 is movable relative to the liquid storing element 320 in the axial direction of the liquid storing element 320 to be switched between a first position and a second position.

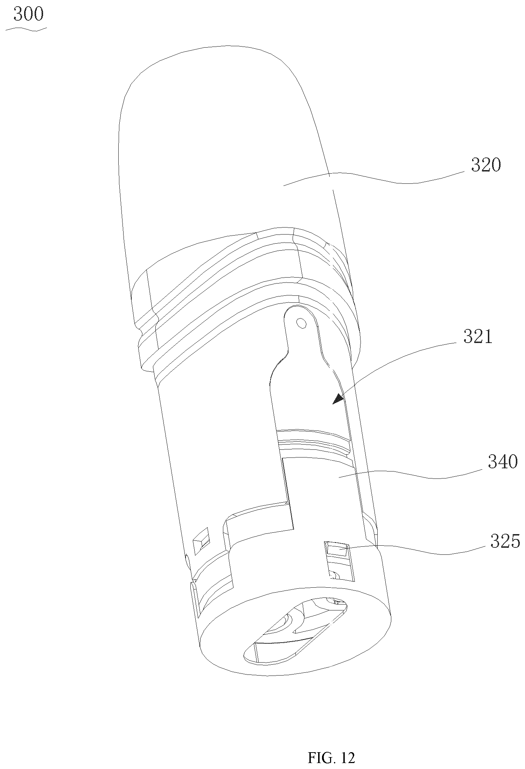

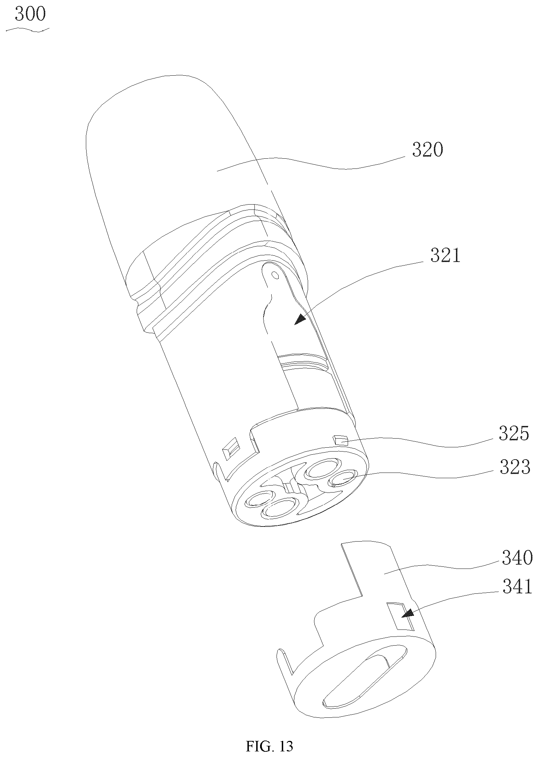

Specifically, an attractive force is provided between the liquid storing element 320 and the protecting element 340. As shown in FIG. 11, when the protecting element 340 is in the first position, the liquid storing element 320 and the protecting element 340 are attracted to each other, the protecting element 340 shields the sealing element 321 to prevent the child from opening the liquid injecting hole. As shown in FIG. 12 and FIG. 13, when the protecting element 340 is in the second position, the protecting element 340 can be disengaged from the liquid storing element 320 by an external force that overcomes the attractive force between the protecting element 340 and the liquid storing element 320, thereby exposing the sealing element 321 to add the atomizing medium.

More specifically, in one embodiment, the bottom of the liquid storing element 320 is embedded with an attracting element 323 formed by a magnet. The protecting element 340 is made of a material that can be attracted by the magnet, such as one or more of iron, cobalt, and nickel. In this way, the liquid storing element 320 can be connected to the protecting element 340 through the attracting element 323.

Referring to FIG. 13, further, in order to prevent the protecting element 340 in the second position from being separated from the liquid storing element 320 and lost, the protecting element 340 is provided with at least one limiting groove 341, and the outer side wall of the liquid storing element 320 is provided with at least one limiting block 325. The limiting groove 341 extends in the axial direction of the liquid storing element 320, the limiting block 325 extends into the limiting groove 341 and can slide along the limiting groove 341. When the protecting element 340 is in the second position, the limiting block 325 abuts against an end wall of the limiting groove 341 and cannot continue to move, thereby keeping the protecting element 340 in the second position, so that the protecting element 340 is kept at the second position.

It should be understood that the positions of the limiting groove 341 and the limiting block 325 are not limited hereto. In other embodiments, the limiting block 325 is provided on the inner sidewall of the protecting element 340, the limiting groove 341 is provided on the outer side wall of the liquid storing element 320, which can also limit the movement of the protecting element 340.

A working principle of the atomizing device 300 will be described as follows.

As shown in FIG. 11, when there is no need to add the atomizing medium, the protecting element 340 and the attracting element 323 at the bottom of the liquid storing element 320 attract each other. At this time, the protecting element 340 is in the first position, the protecting element 340 shields at least part of the sealing element 321 to prevent the child from opening the liquid injecting hole.

As shown in FIG. 12, when adding the atomizing medium, the protecting element 340 is pulled along the axial direction of the liquid storing element 320, the protecting element 340 overcomes the attractive force between the protecting element 340 and the liquid storing element 320 to move to the second position. At this time, the sealing element 321 is completely exposed to the external environment, the operator can open the sealing element 321 to add the atomizing medium through the liquid injecting hole.

After the atomizing medium is added, the pulling force applied on the protecting element 340 is removed, the protecting element 340 can return to the first position under the action of the attractive force of the attracting element 323.

During the above process, the limiting block 325 can slide in the limiting groove 341 to limit the moving direction and the stroke of the protecting element 340.

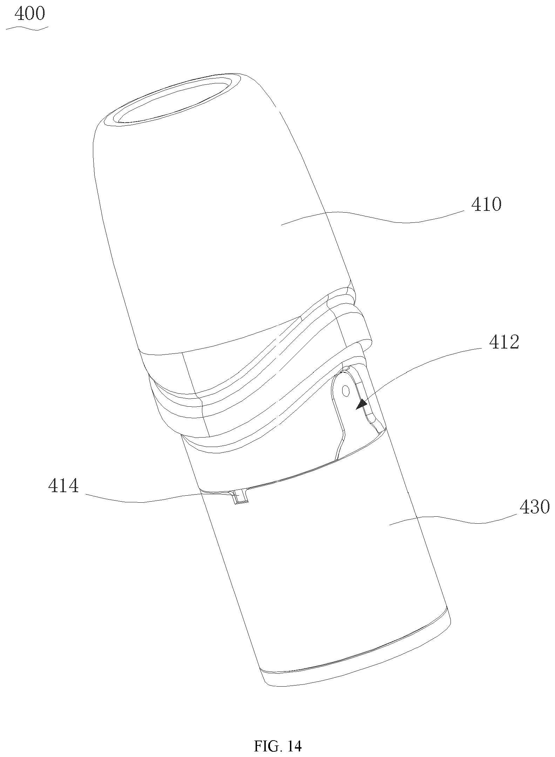

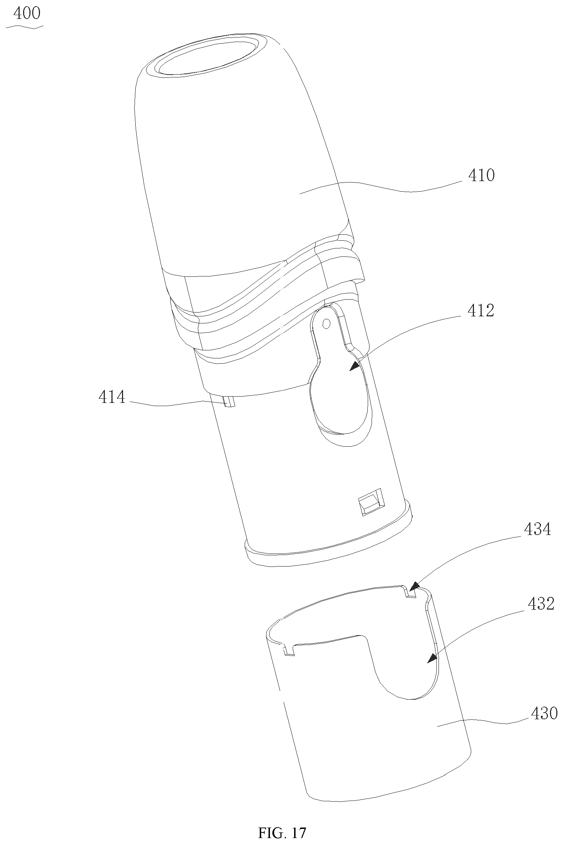

As shown in FIG. 14 to FIG. 17, an atomizing device 400 is provided according to a fourth embodiment of the present disclosure, which includes a liquid storing element 410 and a protecting element 430. An end of the liquid storing element 410 provided with the sealing element 412 is substantially cylindrical, the protecting element 430 has a hollow cylindrical structure and is sleeved on the liquid storing element 410, and a central axis of the protecting element 430 is coincident with a central axis of the liquid storing element 410. The protecting element 430 is movable relative to the liquid storing element 410 in the axial direction of the liquid storing element 410 to be switched between a first position and a second position.

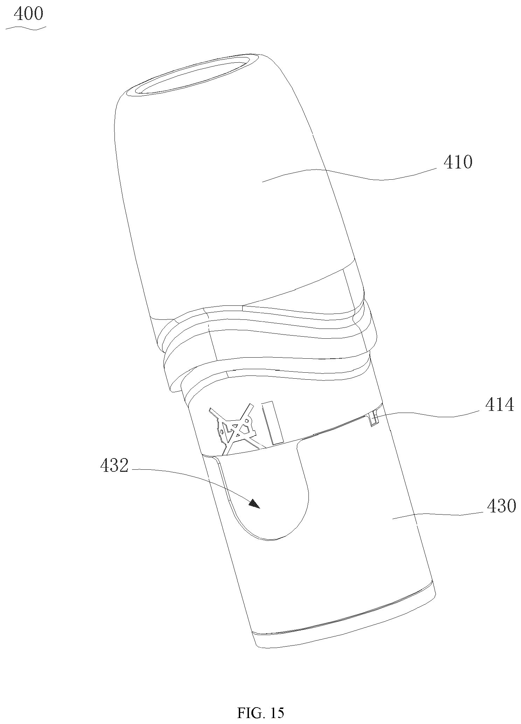

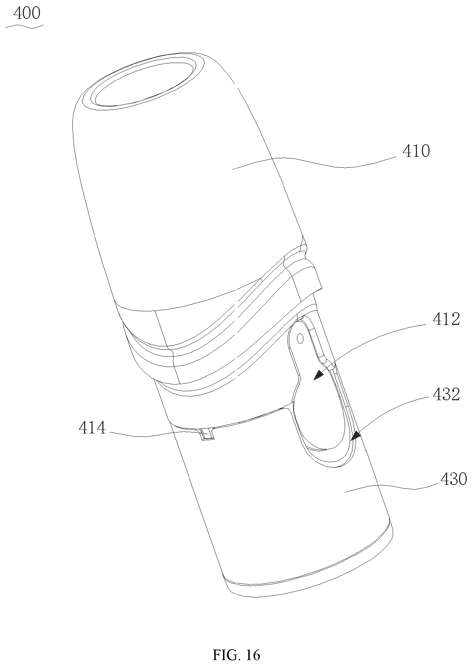

Specifically, an end of the protecting element 430 is provided with a liquid injecting notch 432, and a shape of the liquid injecting notch 432 matches a shape of the sealing element 412. When the protecting element 430 is in the first position (as shown in FIGS. 14 and 15), the liquid injecting notch 432 and the sealing element 412 are misaligned in the circumferential direction of the liquid storing element 410, so that the protecting element 430 shields at least part of the sealing element 412. When the protecting element 430 is in the second position (as shown in FIG. 16), the liquid injecting notch 432 is aligned with the sealing element 412 in the circumferential direction of the liquid storing element 410 to expose the sealing element 412, so that the user can open the sealing element 412 to add the atomizing medium.

Further, an end of the protecting element 430 is provided with at least two engaging notches 434, and the at least two engaging notches 434 are spaced apart in the circumferential direction of the protecting element 430. An outer sidewall of the liquid storing element 410 is provided with an engaging portion 414 that match the engaging notch 434. When the protecting element 430 is switched from the first position to the second position, the engaging portion 414 is engaged in different engaging notches 434, thereby increasing some resistance to the position switching of the protecting element 430 and preventing the child from rotating the protecting element 430. It should be understood that the positions of the engaging notch 434 and the engaging portion 414 are not limited hereto. In other embodiments, the engaging notch 434 can be provided in the liquid storing element 410, the engaging portion 414 can be provided on an inner sidewall of the protecting element 430.

A working principle of the atomizing device 400 will be described as follows.

As shown in FIG. 14 and FIG. 15, when there is no need to add the atomizing medium, the engaging portion 414 is engaged with one of the engaging notches 434. At this time, the protecting element 430 is in the first position, the protecting element 430 shields at least part of the sealing element 412 to prevent the child from opening the liquid injecting hole.

As shown in FIG. 16, when adding the atomizing medium, the protecting element 430 is rotated to generate a recoverable deformation, the engaging portion 414 is disengaged from the current engaging notch 434. When the protecting element 430 is rotated to the second position, the engaging portion 414 is engaged with another engaging notch 434. At this time, the sealing element 412 is completely exposed to the external environment, the user can open the sealing element 412 to add the atomizing medium.

After the atomizing medium is added, the protecting element 430 is rotated to generate a recoverable deformation, the engaging portion 414 is disengaged from the current engaging notch 434. When the protecting element 430 is rotated to the first position again, the engaging portion 414 is engaged in the initial engaging notch 434.

In the above-mentioned atomizing device 400, the protecting element 430 can shield the sealing element 412, which effectively prevents the child from opening the liquid injecting hole to contact the atomizing medium, thereby improving the safety performance of the atomizing device 400 and avoiding the waste of the atomizing medium.

## Claims

1. An atomizing device, comprising:
a liquid storing element (120) provided with a liquid injecting hole;
a sealing element (121) detachably connected to the liquid storing element (120) and configured to seal the liquid injecting hole; and
a protecting element (140) connected to the liquid storing element (120);
wherein the protecting element (140) is connected to the liquid storing element (120), the protecting element (140) is movable relative to the liquid storing element (120) and is capable of switching between a first position and a second position, wherein the shielding area of the sealing element (121) by the protecting element (140) in the first position is greater than the shielding area of the sealing element (121) by the protecting element (140) in the second position;
wherein the protecting element (140) is capable of moving along the axial direction of the liquid storing element (120) relative to the liquid storing element (120); and
**characterised in that**
when the protecting element (140) is in the first position, the protecting element (140) is capable of rotating around the axial direction of the liquid storing element (120) and is capable of switching between a locking state and an unlocking state; when the protecting element (140) is in the locking state, the protecting element (140) is fixed relative to the liquid storing element (120) in the axial direction of the liquid storing element (120); when the protecting element (140) is in the unlocking state, the protecting element (140) is capable of moving in the axial direction of the liquid storing element (120) to be switched to the second position.

2. The atomizing device according to claim 1, wherein at least part of the sealing element (121) is shielded by the protecting element (140) when the protecting element (140) is in the first position, the sealing element (121) is completely exposed to external environment when the protecting element (140) is in the second position.

3. The atomizing device according to claim 1, wherein one of the liquid storing element (120) and the protecting element (140) is provided with a buckle (141), the other one of the liquid storing element (120) and the protecting element (140) is provided with a first guiding groove (123) extending along the circumferential direction of the liquid storing element (120), when the protecting element (140) is in the locking state, the buckle (141) is engaged in the first guiding groove (123).

4. The atomizing device according to claim 3, wherein a transiting portion (125) protrudes within the first guiding groove (123), when the protecting element (140) is in the unlocking state, the buckle (141) abuts against the transiting portion (125) to be disengaged from the first guiding groove (123).

5. The atomizing device according to claim 4, wherein one of the liquid storing element (120) and the protecting element (140) provided with the first guiding groove (123) is further provided with a second guiding groove (127), the second guiding groove (127) is located on a side of the first guiding groove (123) in the axial direction of the liquid storing element (120), when the protecting element (140) is in the second position, the buckle (141) is limited in the second guiding groove (127).

6. The atomizing device according to claim 5, wherein the second guiding groove (127) and the first guiding groove (123) are spaced apart in the axial direction of the liquid storing element (120); or
the second guiding groove (127) is in communication with the first guiding groove (123), and a bottom wall of the second guiding groove (127) is smoothly connected to an outer surface of the transiting portion (125).

7. The atomizing device according to claim 1, wherein the protecting element (140) is provided with an indicating groove (143), when the protecting element (140) is in the locking state, the indicating groove (143) and the sealing element (121) are misaligned in the circumferential direction of the liquid storing element (120), when the protecting element (140) is in the unlocking state, the indicating groove (143) is aligned with the sealing element (121) in the circumferential direction of the liquid storing element (120).

8. The atomizing device according to claim 1, wherein one of the liquid storing element (210) and the protecting element (230) is provided with an engaging protrusion (214), the other one of the liquid storing element (210) and the protecting element (230) is provided with an engaging groove (232) and a sliding groove (234), the engaging groove (232) and the sliding groove (234) are spaced apart in the circumferential direction of the liquid storing element (210), and the length of the sliding groove (234) is greater than the length of the engaging groove (232) in the axial direction of the liquid storing element (210), when the protecting element (230) is in the locking state, the engaging protrusion (214) is engaged in the engaging groove (232), when the protecting element (230) is in the unlocking state, the engaging protrusion (214) is located in the sliding groove (234).

9. The atomizing device according to claim 1, wherein an attractive force is provided between the liquid storing element (320) and the protecting element (340), when the protecting element (340) is in the first position, the liquid storing element (320) and the protecting element (340) are attracted to each other, when the protecting element (340) is in the second position, the protecting element (340) is disengaged from the liquid storing element (320) by an external force that overcomes the attractive force.

10. The atomizing device according to claim 9, wherein one of the liquid storing element (320) and the protecting element (340) is provided with a limiting groove (341), the other one of the liquid storing element (320) and the protecting element (340) is provided with a limiting block (325), the limiting groove (341) extends in the axial direction of the liquid storing element (320) or the protecting element (340), the limiting block (325) extends into the limiting groove (341) and is capable of sliding along the limiting groove (341).

11. The atomizing device according to claim 1, wherein the protecting element (430) is rotatable relative to the liquid storing element (410) about the axial direction of the liquid storing element (410).

12. The atomizing device according to claim 11, wherein the protecting element (430) is provided with a liquid injecting notch (432), when the protecting element (430) is in the first position, the liquid injecting notch (432) and the sealing element (412) are misaligned, when the protecting element (430) is in the second position, the liquid injecting notch (432) is aligned with the sealing element (412) to expose the sealing element (412).

13. The atomizing device according to claim 11, wherein one of the liquid storing element (410) and the protecting element (430) is provided with at least two engaging notches (434), the at least two engaging notches (434) are spaced apart in the circumferential direction of the protecting element (430), the other one of the liquid storing element (410) and the protecting element (430) is provided with an engaging portion (414), the engaging portion (414) is engaged in one of the engaging notches (434) when the protecting element (430) is in the first position, and the engaging portion (414) is engaged in another engaging notch (434) when the protecting element (430) is rotated to the second position.

## Patentansprüche

1. Zerstäubungsvorrichtung, aufweisend:
ein mit einer Flüssigkeitseinfüllöffnung versehenes Flüssigkeitsspeicherelement (120);
ein Abdichtelement (121), das abnehmbar mit dem Flüssigkeitsspeicherelement (120) verbunden ist und dazu eingerichtet ist, die Flüssigkeitseinfüllöffnung abzudichten; und
ein mit dem Flüssigkeitsspeicherelement (120) verbundenes Schutzelement (140);
wobei das Schutzelement (140) mit dem Flüssigkeitsspeicherelement (120) verbunden ist, das Schutzelement (140) relativ zu dem Flüssigkeitsspeicherelement (120) beweglich ist und in der Lage ist, zwischen einer ersten Position und einer zweiten Position zu wechseln, wobei der Abschirmbereich des Abdichtelements (121) durch das Schutzelement (140) in der ersten Position größer ist als der Abschirmbereich des Abdichtelements (121) durch das Schutzelement (140) in der zweiten Position;
wobei das Schutzelement (140) in der Lage ist, sich entlang der Axialrichtung des Flüssigkeitsspeicherelements (120) relativ zu dem Flüssigkeitsspeicherelement (120) zu bewegen; und
**dadurch gekennzeichnet, dass**, wenn sich das Schutzelement (140) in der ersten Position befindet, das Schutzelement (140) in der Lage ist, sich um die Axialrichtung des Flüssigkeitsspeicherelements (120) zu drehen, und zwischen einem Verriegelungszustand und einem Entriegelungszustand umschaltbar ist; wenn sich das Schutzelement (140) in dem Verriegelungszustand befindet, das Schutzelement (140) relativ zu dem Flüssigkeitsspeicherelement (120) in der Axialrichtung des Flüssigkeitsspeicherelements (120) fixiert ist; wenn sich das Schutzelement (140) in dem Entriegelungszustand befindet, das Schutzelement (140) in der Lage ist, sich in der Axialrichtung des Flüssigkeitsspeicherelements (120) zu bewegen, um in die zweite Position zu wechseln.

2. Zerstäubungsvorrichtung nach Anspruch 1, wobei zumindest ein Teil des Abdichtelements (121) durch das Schutzelement (140) abgeschirmt ist, wenn sich das Schutzelement (140) in der ersten Position befindet, und das Abdichtelement (121) vollständig der Außenumgebung ausgesetzt ist, wenn sich das Schutzelement (140) in der zweiten Position befindet.

3. Zerstäubungsvorrichtung nach Anspruch 1, wobei ein Element aus dem Flüssigkeitsspeicherelement (120) und dem Schutzelement (140) mit einer Schnalle (141) versehen ist, das andere Element aus dem Flüssigkeitsspeicherelement (120) und dem Schutzelement (140) mit einer ersten Führungsnut (123) versehen ist, die sich entlang der Umfangsrichtung des Flüssigkeitsspeicherelements (120) erstreckt, und die Schnalle (141), wenn sich das Schutzelement (140) in dem Verriegelungszustand befindet, in die erste Führungsnut (123) eingreift.

4. Zerstäubungsvorrichtung nach Anspruch 3, wobei ein Übergangsabschnitt (125) innerhalb der ersten Führungsnut (123) vorsteht und die Schnalle (141), wenn sich das Schutzelement (140) in dem Entriegelungszustand befindet, an dem Übergangsabschnitt (125) anliegt, um von der ersten Führungsnut (123) gelöst zu werden.

5. Zerstäubungsvorrichtung nach Anspruch 4, wobei das Element aus dem Flüssigkeitsspeicherelement (120) und dem Schutzelement (140), das mit der ersten Führungsnut (123) versehen ist, ferner mit einer zweiten Führungsnut (127) versehen ist, die zweite Führungsnut (127) auf einer Seite der ersten Führungsnut (123) in der Axialrichtung des Flüssigkeitsspeicherelements (120) angeordnet ist und die Schnalle (141), wenn sich das Schutzelement (140) in der zweiten Position befindet, in der zweiten Führungsnut (127) begrenzt ist.

6. Zerstäubungsvorrichtung nach Anspruch 5, wobei die zweite Führungsnut (127) und die erste Führungsnut (123) in der Axialrichtung des Flüssigkeitsspeicherelements (120) beabstandet sind; oder die zweite Führungsnut (127) mit der ersten Führungsnut (123) in Verbindung steht und eine Bodenwand der zweiten Führungsnut (127) glatt mit einer Außenfläche des Übergangsabschnitts (125) verbunden ist.

7. Zerstäubungsvorrichtung nach Anspruch 1, wobei das Schutzelement (140) mit einer Anzeigenut (143) versehen ist, die Anzeigenut (143) und das Abdichtelement (121), wenn sich das Schutzelement (140) in dem Verriegelungszustand befindet, in der Umfangsrichtung des Flüssigkeitsspeicherelements (120) versetzt sind und die Anzeigenut (143), wenn sich das Schutzelement (140) in dem Entriegelungszustand befindet, in der Umfangsrichtung des Flüssigkeitsspeicherelements (120) mit dem Abdichtelement (121) fluchtet.

8. Zerstäubungsvorrichtung nach Anspruch 1, wobei ein Element aus dem Flüssigkeitsspeicherelement (210) und dem Schutzelement (230) mit einem Eingriffsvorsprung (214) versehen ist, das andere Element aus dem Flüssigkeitsspeicherelement (210) und dem Schutzelement (230) mit einer Eingriffsnut (232) und einer Gleitnut (234) versehen ist, die Eingriffsnut (232) und die Gleitnut (234) in der Umfangsrichtung des Flüssigkeitsspeicherelements (210) beabstandet sind, die Länge der Gleitnut (234) größer ist als die Länge der Eingriffsnut (232) in der Axialrichtung des Flüssigkeitsspeicherelements (210), der Eingriffsvorsprung (214), wenn sich das Schutzelement (230) in dem Verriegelungszustand befindet, in die Eingriffsnut (232) eingreift und der Eingriffsvorsprung (214), wenn sich das Schutzelement (230) in dem Entriegelungszustand befindet, in der Gleitnut (234) angeordnet ist.

9. Zerstäubungsvorrichtung nach Anspruch 1, wobei eine Anziehungskraft zwischen dem Flüssigkeitsspeicherelement (320) und dem Schutzelement (340) vorgesehen ist, das Flüssigkeitsspeicherelement (320) und das Schutzelement (340), wenn sich das Schutzelement (340) in der ersten Position befindet, einander anziehen und das Schutzelement (340), wenn sich das Schutzelement (340) in der zweiten Position befindet, durch eine äußere Kraft, die die Anziehungskraft überwindet, von dem Flüssigkeitsspeicherelement (320) gelöst ist.

10. Zerstäubungsvorrichtung nach Anspruch 9, wobei ein Element aus dem Flüssigkeitsspeicherelement (320) und dem Schutzelement (340) mit einer Begrenzungsnut (341) versehen ist, das andere Element aus dem Flüssigkeitsspeicherelement (320) und dem Schutzelement (340) mit einem Begrenzungsblock (325) versehen ist, die Begrenzungsnut (341) sich in der Axialrichtung des Flüssigkeitsspeicherelements (320) oder des Schutzelements (340) erstreckt und der Begrenzungsblock (325) sich in die Begrenzungsnut (341) erstreckt und in der Lage ist, entlang der Begrenzungsnut (341) zu gleiten.

11. Zerstäubungsvorrichtung nach Anspruch 1, wobei das Schutzelement (430) relativ zu dem Flüssigkeitsspeicherelement (410) um die Axialrichtung des Flüssigkeitsspeicherelements (410) drehbar ist.

12. Zerstäubungsvorrichtung nach Anspruch 11, wobei das Schutzelement (430) mit einer Flüssigkeitseinfüllkerbe (432) versehen ist, die Flüssigkeitseinfüllkerbe (432) und das Abdichtelement (412), wenn sich das Schutzelement (430) in der ersten Position befindet, versetzt sind und die Flüssigkeitseinfüllkerbe (432), wenn sich das Schutzelement (430) in der zweiten Position befindet, mit dem Abdichtelement (412) ausgerichtet ist, um das Abdichtelement (412) freizulegen.

13. Zerstäubungsvorrichtung nach Anspruch 11, wobei ein Element aus dem Flüssigkeitsspeicherelement (410) und dem Schutzelement (430) mit mindestens zwei Eingriffskerben (434) versehen ist, die mindestens zwei Eingriffskerben (434) in der Umfangsrichtung des Schutzelements (430) beabstandet sind, das andere Element aus dem Flüssigkeitsspeicherelement (410) und dem Schutzelement (430) mit einem Eingriffsabschnitt (414) versehen ist, der Eingriffsabschnitt (414), wenn sich das Schutzelement (430) in der ersten Position befindet, in eine der Eingriffskerben (434) eingreift und der Eingriffsabschnitt (414), wenn das Schutzelement (430) in die zweite Position gedreht wird, in eine andere Eingriffskerbe (434) eingreift.

## Revendications

1. Dispositif de pulvérisation, comprenant:
un élément de stockage de liquide (120) pourvu d'un orifice d'injection de liquide;
un élément d'étanchéité (121) connecté de manière amovible à l'élément de stockage de liquide (120) et configuré pour fermer hermétiquement l'orifice d'injection de liquide; et
un élément de protection (140) connecté à l'élément de stockage de liquide (120);
dans lequel l'élément de protection (140) est connecté à l'élément de stockage de liquide (120), l'élément de protection (140) est mobile par rapport à l'élément de stockage de liquide (120) et est capable de basculer entre une première position et une deuxième position, dans lequel la zone de blindage de l'élément d'étanchéité (121) par l'élément de protection (140) dans la première position est supérieure à la zone de blindage de l'élément d'étanchéité (121) par l'élément de protection (140) dans la deuxième position;
dans lequel l'élément de protection (140) est capable de se déplacer le long de la direction axiale de l'élément de stockage de liquide (120) par rapport à l'élément de stockage de liquide (120); et
**caractérisé en ce que**
lorsque l'élément de protection (140) se trouve dans la première position, l'élément de protection (140) est capable de tourner autour de la direction axiale de l'élément de stockage de liquide (120) et est capable de basculer entre un état de verrouillage et un état de déverrouillage; lorsque l'élément de protection (140) se trouve dans l'état de verrouillage, l'élément de protection (140) est fixe par rapport à l'élément de stockage de liquide (120) dans la direction axiale de l'élément de stockage de liquide (120); lorsque l'élément de protection (140) se trouve dans l'état de déverrouillage, l'élément de protection (140) est capable de se déplacer dans la direction axiale de l'élément de stockage de liquide (120) pour être basculé vers la deuxième position.

2. Dispositif de pulvérisation selon la revendication 1, dans lequel au moins une partie de l'élément d'étanchéité (121) est protégée par l'élément de protection (140) lorsque l'élément de protection (140) se trouve dans la première position, l'élément d'étanchéité (121) est complètement exposé à l'environnement extérieur lorsque l'élément de protection (140) se trouve dans la deuxième position.

3. Dispositif de pulvérisation selon la revendication 1, dans lequel l'un parmi l'élément de stockage de liquide (120) et l'élément de protection (140) est pourvu d'une boucle (141), l'autre parmi l'élément de stockage de liquide (120) et l'élément de protection (140) est pourvu d'une première rainure de guidage (123) s'étendant le long de la direction circonférentielle de l'élément de stockage de liquide (120), lorsque l'élément de protection (140) se trouve dans l'état de verrouillage, la boucle (141 123) est engagée dans la première rainure de guidage.

4. Dispositif de pulvérisation selon la revendication 3, dans lequel une partie de passage (125) fait saillie à l'intérieur de la première rainure de guidage (123), lorsque l'élément de protection (140) se trouve dans l'état de déverrouillage, la boucle (141) vient en butée contre la partie de passage (125) pour être désengagée de la première rainure de guidage (123).

5. Dispositif de pulvérisation selon la revendication 4, dans lequel l'un parmi l'élément de stockage de liquide (120) et l'élément de protection (140) pourvu de la première rainure de guidage (123) est en outre pourvu d'une deuxième rainure de guidage (127), la deuxième rainure de guidage (127) est située sur un côté de la première rainure de guidage (123) dans la direction axiale de l'élément de stockage de liquide (120), lorsque l'élément de protection (140) se trouve dans la deuxième position, la boucle (141) est limitée dans la deuxième rainure de guidage (127).

6. Dispositif de pulvérisation selon la revendication 5, dans lequel la deuxième rainure de guidage (127) et la première rainure de guidage (123) sont espacées l'une de l'autre dans la direction axiale de l'élément de stockage de liquide (120); ou
la deuxième rainure de guidage (127) est en communication avec la première rainure de guidage (123), et une paroi inférieure de la deuxième rainure de guidage (127) est connectée de manière régulière à une surface extérieure de la partie de passage (125).

7. Dispositif de pulvérisation selon la revendication 1, dans lequel l'élément de protection (140) est pourvu d'une rainure d'indication (143), lorsque l'élément de protection (140) se trouve dans l'état de verrouillage, la rainure d'indication (143) et l'élément d'étanchéité (121) sont désalignés dans la direction circonférentielle de l'élément de stockage de liquide (120), lorsque l'élément de protection (140) est dans l'état de déverrouillage, la rainure d'indication (143) est alignée avec l'élément d'étanchéité (121) dans la direction circonférentielle de l'élément de stockage de liquide (120).

8. Dispositif de pulvérisation selon la revendication 1, dans lequel l'un parmi l'élément de stockage de liquide (210) et l'élément de protection (230) est pourvu d'une saillie d'engagement (214), l'autre parmi l'élément de stockage de liquide (210) et l'élément de protection (230) est pourvu d'une rainure d'engagement (232) et d'une rainure de coulissement (234), la rainure d'engagement (232) et la rainure de coulissement (234) sont espacées l'une de l'autre dans la direction circonférentielle de l'élément de l'élément de stockage de liquide (210), et la longueur de la rainure de coulissement (234) est supérieure à la longueur de la rainure d'engagement (232) dans la direction axiale de l'élément de stockage de liquide (210), lorsque l'élément de protection (230) est dans l'état de verrouillage, la saillie d'engagement (214) est engagée dans la rainure d'engagement (232), lorsque l'élément de protection (230) est dans l'état de déverrouillage, la saillie d'engagement (214) est située dans la rainure de coulissement (234).

9. Dispositif de pulvérisation selon la revendication 1, dans lequel une force d'attraction est fournie entre l'élément de stockage de liquide (320) et l'élément de protection (340), lorsque l'élément de protection (340) se trouve dans la première position, l'élément de stockage de liquide (320) et l'élément de protection (340) sont attirés l'un vers l'autre, lorsque l'élément de protection (340) se trouve dans la deuxième position, l'élément de protection (340) est désengagé de l'élément de stockage de liquide (320) par une force externe qui surmonte la force d'attraction.

10. Dispositif de pulvérisation selon la revendication 9, dans lequel l'un parmi l'élément de stockage de liquide (320) et l'élément de protection (340) est pourvu d'une rainure de limitation (341), l'autre parmi l'élément de stockage de liquide (320) et l'élément de protection (340) est pourvu d'un bloc de limitation (325), la rainure de limitation (341) s'étendant dans la direction axiale de l'élément de stockage de liquide (320) ou de l'élément de protection (340), le bloc de limitation (325) s'étend dans la rainure de limitation (341) et est capable de coulisser le long de la rainure de limitation (341).

11. Dispositif de pulvérisation selon la revendication 1, dans lequel l'élément de protection (430) peut tourner par rapport à l'élément de stockage de liquide (410) autour de la direction axiale de l'élément de stockage de liquide (410).

12. Dispositif de pulvérisation selon la revendication 11, dans lequel l'élément de protection (430) est pourvu d'une encoche d'injection de liquide (432), lorsque l'élément de protection (430) se trouve dans la première position, l'encoche d'injection de liquide (432) et l'élément d'étanchéité (412) sont désalignés, lorsque l'élément de protection (430) se trouve dans la deuxième position, l'encoche d'injection de liquide (432) est alignée avec l'élément d'étanchéité (412) pour exposer l'élément d'étanchéité (412).

13. Dispositif de pulvérisation selon la revendication 11, dans lequel l'un parmi l'élément de stockage de liquide (410) et l'élément de protection (430) est pourvu d'au moins deux encoches d'engagement (434), lesdites au moins deux encoches d'engagement (434) sont espacées l'une de l'autre dans la direction circonférentielle de l'élément de protection (430), l'autre parmi l'élément de stockage de liquide (410) et l'élément de protection (430) est pourvu d'une partie d'engagement (414), la partie d'engagement (414) est engagée dans l'une des encoches d'engagement (434) lorsque l'élément de protection (430) se trouve dans la première position, et la partie d'engagement (414) est engagée dans une autre encoche d'engagement (434) lorsque l'élément de protection (430) est tourné vers la deuxième position.
